# EUROPEAN PATENT SPECIFICATION

(11) **EP 3 684 380 B1**
(45) Date of publication and mention of the grant of the patent: **08.09.2021**
(21) Application number: 18789505.7
(22) Date of filing: 20.09.2018
(51) Int. Cl.: A61K 33/04, A61K 33/22, A61K 8/19, A61K 8/23, A61K 9/00, A61K 47/02, A61K 47/10, A61P 17/04, A61P 17/10, A61P 17/06, A61P 17/00, A61Q 19/00

(54) **AQUEOUS FORMULATION FOR THE TOPICAL TREATMENT OF SKIN CONDITIONS**
WÄSSRIGE FORMULIERUNG ZUR TOPISCHEN BEHANDLUNG VON HAUTERKRANKUNGEN
FORMULATION AQUEUSE POUR LE TRAITEMENT TOPIQUE D'AFFECTIONS CUTANÉES

(30) Priority: 21.09.2017 NL 2019602
(43) Date of publication of application: 29.07.2020
(73) Proprietor: Rosaderma B.V., 7895 AP Roswinkel (NL)
(72) Inventor: KAIJSER-GRIGORIANTS, Rozitta, 7895 AP Roswinkel (NL)
(74) Representative: Nederlandsch Octrooibureau
(86) International application number: PCT/NL2018/050622
(87) International publication number: WO 2019/059766

(56) References cited:
- WO-A1-2008/140200
- DATABASE WPI Week 199724 Thomson Scientific, London, GB; AN 1997-259980 XP002781001, & CN 1 101 552 A (HE Z) 19 April 1995 (1995-04-19)
- DATABASE WPI Week 201154 Thomson Scientific, London, GB; AN 2011-F22077 XP002781002, & CN 102 000 141 A (QI X) 6 April 2011 (2011-04-06)
- DATABASE WPI Week 201755 Thomson Scientific, London, GB; AN 2017-41605R XP002781003, & CN 106 821 930 A (ZHOU G) 13 June 2017 (2017-06-13)
- MUHAMMAD ATHAR ABBASI ET AL: "Preparation of new formulations of anti-acne creams and their efficacy", AFRICAN JOURNAL OF PHARMACY AND PHARMACOLOGY, vol. 4, 1 June 2010 (2010-06-01), pages 298-303, XP055473496,
- Anonymous: "Sulfur / Borax - Uses, Side-effects, Reviews, and Precautions", TabletWise, 7 May 2017 (2017-05-07), pages 1-4, XP055473332, Retrieved from the Internet: URL:https://www.tabletwise.com/medicine/su lfur-borax [retrieved on 2018-05-08]

## Description

### TECHNICAL FIELD OF THE INVENTION

The present invention relates to aqueous formulations for the topical treatment of skin conditions, as defined in the claims. In particular, the invention relates to formulations containing borax and sulphur, as defined in the claims.

### BACKGROUND OF THE INVENTION

Skin conditions affect millions of men and women throughout the world, mostly between the age of 20 to 60 years. Amongst the most common chronic skin conditions in adult life are rosacea, acne, and eczema.

Rosacea in particular is a common inflammatory skin disease manifested in 4 subtypes, and characterized by flushing and persistent redness in the cheeks, nose, chin, and forehead. If untreated, some subtypes of rosacea may result in permanent enlargement of the nose from excess tissue and/or potential vision loss from corneal damage. Nevertheless, it is the aesthetic aspect of rosacea and other similar chronic skin diseases that mostly often result in intense psychosocial distress of the affected subjects.

WO2008/140200 describes external compositions comprising dissolved sulphur and alum for curing or relieving skin diseases, where the skin diseases to be treated by the composition can be dermal inflammations, acne, xerosis, tinea pedis, eczema, allergic rushes, pruritus, tinea, psoriasis, pustulosis palmaris et plantaris, or atopic diseases. Composition SBCO-1 comprises sulphur, alum, camphor, menthol, silver, borax *Phellodendron amurensis.* 0.15g sulphur was dissolved in 5mL alkaline aqueous solution (pH12-13.5) or a potassium hydroxide solution. 0.2g borax was dissolved in 5mL water.

CN105434299A describes an acne facial mask cream comprising 15-20% of alocasia macrorrhiza, 3-5% of sophora flavescens alkaloid, 6-10% of dried Chinese gall extract, 35-45 % of dried alum, 20-30 % of sulphur and 10-20 % of calcined borax. The cream is prepared by uniformly mixing the components, adding 1000ml of water, stirring for 5-10 minutes, and uniformly mixing; then, filtering with gauze, uniformly mixing the medicinal liquor with filtered coix seed powder in a mass ratio of 3:1 to obtain the acne facial mask cream; followed by packaging.

CN1101552A discloses the treatment of tinea versicolor with a composition obtained by mixing sulfur, borax and zinc oxide, grinding them into a powder, mixing the powder with cold, boiled water, and applying the obtained composition to the affected body parts.

There remains a need for a simple, easy to use topical formulation for the treatment of skin conditions such as rosacea.

### SUMMARY OF THE INVENTION

The inventors have unexpectedly found that symptoms associated with skin conditions such as rosacea and acne can be ameliorated by topically applying an aqueous formulation comprising undissolved borax and sulphur particles.

Thus, in a first aspect, the present invention relates to an aqueous formulation for the topical treatment of skin conditions, comprising:
a) 2-30 parts by weight undissolved borax particles;
b) 0.01-10 parts by weight undissolved sulphur particles; and
c) 20-95 parts by weight water, wherein the ratio of undissolved borax particles to undissolved sulphur particles is in the range of 20:1 to 1.5:1 at 20 °C.

It has been found that the aqueous formulation of the present invention can be used to reduce symptoms such as reddish, blotchy skin and pustules, so that the skin has a healthy, radiant appearance devoid of reddish, blotchy skin and pustules. Unexpectedly, when skin is treated with the aqueous formulation, symptoms associated with skin conditions such as rosacea and acne can be ameliorated so that symptoms do not return 6 months after treatment with the formulation.

In a second aspect, the present invention relates to a method for preparing an aqueous formulation comprising the steps of mixing borax powder, sulphur powder and water at a temperature of not more than 30 °C.

It has been surprisingly found that when the borax and sulphur particles are mixed by shaking or otherwise agitated at room temperature in water, an aqueous formulation of undissolved borax and sulphur particles is obtained that is particularly effective at treating skin conditions such as rosacea and acne.

### DETAILED DESCRIPTION OF THE INVENTION

In a first aspect, the present invention relates to an aqueous formulation for the topical treatment of skin conditions, comprising:
a) 2-30 parts by weight undissolved borax particles;
b) 0.01-10 parts by weight undissolved sulphur particles; and
c) 20-95 parts by weight water, wherein the ratio of undissolved borax particles to undissolved sulphur particles is in the range of 20:1 to 1.5:1 at 20 °C.

The term "skin condition" as used herein refers to dermatologic conditions that exhibit a undesirable, unsightly skin appearance. Such conditions are often caused by inflammatory, bacterial, fungal or viral means.

The term "borax" as used herein refers to sodium borate. The sodium borate may by either hydrous (e.g. Na₂B₄O₇·10H₂O or Na₂B₄O₇·5H₂O) or anhydrous (B₄O₇Na₂).

The term "undissolved borax particles" means that the borax particles are present as solid particles in the aqueous formulation and do not dissolve when the aqueous formulation is kept at 20°C for 1 week.

The term "sulphur" as used herein refers to elemental sulphur (S₈).

The term "undissolved sulphur particles" means that the sulphur particles are present as solid particles in the aqueous formulation and do not dissolve when the aqueous formulation is kept at 20°C for 1 week.

The term "weight ratio of solids" means the ratio of undissolved borax particles to undissolved sulphur particles.

The concentration of undissolved borate particles and undissolved sulphur particles in the aqueous formulation is temperature-dependent. Whenever reference is made to the composition of the aqueous formulation, unless indicated otherwise, it refers to the composition at 20°C. The aqueous formulation of the present invention can be liquid or semi-solid at 20°C.

The borax particles contained in the aqueous formulation of the present invention preferably contain at least 50 wt.%, more preferably at least 90 wt.% and most preferably at least 85 wt.% Na₂B₄O₇, 10H₂O. Preferably, the borax particles comprise more than 99 wt.% Na₂B₄O₇,10H₂O. The sulphur particles contained in the aqueous formulation of the present invention preferably contain at least 50 wt.%, more preferably at least 90 wt.% and most preferably at least 85 wt.% sulphur.

According to the invention, the ratio of undissolved borax particles to undissolved sulphur particles is in the range of 20:1 to 1.5:1 at 20°C, preferably 15:1 to 2:1, more preferably 12:1:3:1, even more preferably 10:1 to 4:1. It has been found that the aqueous formulation is particularly effective when the undissolved borax particles are present in excess, relative to the undissolved sulphur particles. Advantageously, the inventors have found that by incorporating sulphur in the form of undissolved particles the unpleasant odour associated with sulphur-comprising formulations is unexpectedly reduced.

Preferably, the aqueous formulation comprises 50-97.5 wt.% borax particles, based on dry solids, more preferably 60-95 wt.% borax particles, even more preferably 65-90 wt.% borax particles.

In a preferred embodiment, the aqueous formulation comprises 2.5-30 wt.% undissolved sulphur particles, based on dry solids, more preferably 5-25 wt.% sulphur particles, even more preferably 10-20% sulphur particles.

Preferably, the combination of undissolved borax particles and undissolved sulphur particles represents 5-25 wt.% of the total weight of the formulation, more preferably 7.5-20 wt.%, even more preferably 10-15 wt.%.

Typically, the total dry solids of the formulation is 5-50 wt.% of the total weight of the formulation, preferably 10-35 wt.%, even more preferably 15-20 wt.%.

In yet another preferred embodiment, at least 80 wt.%, preferably at least 85 wt.%, and even more preferably at least 90 wt.% of the undissolved borax particles has a particle size of between 50-3000 µm, preferably 100-1200 µm, more preferably 200-1000 µm. It has been found that when the particle size is relatively large (i.e. above 50 µm, preferably above 100 µm and even more preferably above 200 µm) the undissolved borax particles impart a granular texture to the suspension which enables the formulation to effectively coat (i.e. adhere) to the skin.

Preferably, at least 80 wt.%, preferably at least 85 wt.%, and even more preferably at least 90 wt.% of the undissolved sulphur particles has a particle size of 0.0050-5 µm, preferably 0.010-1 µm, more preferably 0.05-0.5 µm.

The particle size distribution of the borax and sulphur particles can be suitably determined using a set of sieves of different mesh sizes. Alternatively, the particle size distribution of the borax and sulphur can be determined, for instance, by means of laser diffraction. Care should be taken that loose aggregates of particles are dissociated before the particle size measurements.

Typically, the weight ratio of solids (i.e. undissolved borax and sulphur particles) to liquids is 1:8 to 1:1, preferably 1:6 to 1:1, more preferably 1:4 to 1:1.

The aqueous formulation may comprise an aqueous phase comprising water and a water miscible organic solvent. Preferably, the aqueous formulation comprises at least 30 parts by weight water, more preferably at least 40 parts by weight water, even more preferably at least 50 parts by weight water, most preferably at least 60 parts by weight water.

Preferably, the aqueous formulation comprises 5-50 parts by weight of the total formulation of a water miscible organic solvent. In a further preferred embodiment, the aqueous formulation comprises 6-40 parts by weight, more preferably 10-35 parts by weight of a water miscible organic solvent.

In a preferred embodiment, the combination of water and water miscible organic solvent constitutes at least 60 wt.%, preferably at least 75 wt.%, of the aqueous formulation.

A suitable water miscible organic solvent is selected from the group consisting of ethanol, ethylene glycol propylene glycol, glycerol and mixtures thereof. Preferably, the water miscible organic solvent is ethanol.

In another preferred embodiment, the combination of undissolved borax particles, undissolved sulphur particles, water and water miscible organic solvent constitutes at least 80 wt.%, preferably at least 90 wt.% of the aqueous formulation.

Particularly preferred is an aqueous formulation comprising, based on total weight of the formulation:
a) 5-15 wt.% undissolved borax particles;
b) 0.1-10 wt.% undissolved sulphur particles; and
c) 50-70 wt.% water; and
d) 5-35 wt.% water miscible organic solvent, wherein the ratio of solids (undissolved borax particles : undissolved sulphur particles) is at least 20:1 to 1.5:1.

Preferably, the formulation comprises 0.001-5 parts by weight dermatologically acceptable terpene selected from the group consisting of menthol, camphor, pinene, thujone, limonene and mixtures thereof. It has been found that these terpenes not only provide the formulation with an acceptable aroma, but such terpenes may further enhance the efficacy of the formulation.

In an embodiment, the aqueous formulation is a liquid or semi-solid selected from gel, lotion, cream or a suspension. The formulation may be one of many topical formulation types containing water as the major ingredient, including suspensions, gels, creams, and lotions. It is preferred, although not required, that the formulation is in the form of an aqueous suspension.

Accordingly, the formulation of the invention may contain an agent to stabilize the suspension such as a gelling or thickening agent. Any gelling agent that is water-dispersible, is suitable for use on epithelial tissue such as skin, and forms an aqueous gel of substantially uniform consistency, is suitable for use in the composition of the invention. One preferred gelling agent is hydroxypropylcellulose, such as that sold under the tradename KLUCEL® (Hercules Incorporated, Wilmington, DE, USA) Another preferred gelling agent is hydroxyethylcellulose, such as that sold under the tradename NATROSOL® (Hercules Incorporated). Other suitable gelling agents include carboxyvinyl polymers, also known as carbomers, such as are sold under the tradename CARBOPOL® 934, 940, 941, 980, and 981 (B. F. Goodrich Co., Akron, OH, USA), ETD 2020™, and ULTREZ® (Noveon, Inc., Cleveland, OH, USA). Additional suitable gelling agents are polyvinyl alcohol, polyethylene oxides, propylene glycol alginates, methylcellulose, hydroxypropylmethylcellulose and natural polymeric gums such as xanthan, and carrageenan. The concentration of gelling agent in the composition may be varied depending on several factors, including the desired degree of stabilization of the BPO suspension and desired viscosity of the gel composition.

In a preferred embodiment, but not necessarily, the formulation of the invention contains at least one additional chemical compound that is effective in the treatment of skin condition. The additional chemical compound may be suspended or dissolved in the formulation.

Preferably, the additional chemical compound is soluble in water and is dissolved in the formulation. One such preferred additional chemical compound is an antibiotic. Preferred antibiotics include those of the macrolide family of antibiotics such as erythromycin, azithromycin, clarithromycin, tilmicosin, and tylosin, and those of the lincomycin family of antibiotics such as clindamycin and lincomycin. Alternatively, additional chemical compound compounds such as sulfamethoxazole and trimethoprim may be included in the formulation.

Additional topical additional chemical compounds that may be contained in the formulation of the invention, either with or without the inclusion of an antibiotic, include miconazole nitrate, salicylic acid, azelaic acid, niacinamide, urea, and retinoids such as tretinoin, adapalene and tazarotene.

If desired, the formulation of the invention may further include additional dermatologically acceptable excipients typically used in formulations and known to those skilled in the art. Such excipients include, for example, humectants, emollients, pH stabilizing agents, preservatives, chelating agents, and anti-oxidants.

The preferred substances, amounts, and ranges etc.. thereof for the first aspect apply mutatis mutandis to the second, third and fifth aspect of the present invention, unless specified otherwise.

In a second aspect, the present invention relates to a method for preparing an aqueous formulation according to the invention comprising the steps of mixing borax particles, sulphur particles, water and the optional water miscible organic solvent at a temperature of not more than 30 °C.

Preferably, the particles are mixed by any conventional means, such as shaking, beating, stirring, air injection, circulation, etc. More preferably, the particles are mixed by vigorous shaking for at least 10 minutes, preferably at least 20 minutes, more preferably at least 30 minutes, most preferably at least 60 minutes. Alternatively, the particles may be mixed by stirring preferably at 100 - 1000 rpm, more preferably at 200 - 800 rpm, even more preferably at 300 - 500 rpm, for 2-30 minutes, preferably 5-25 minutes, more preferably 10-20 minutes.

In a third aspect, the present invention relates to an aqueous formulation as defined herein for use in the treatment of a skin condition, said use comprising the step of applying the aqueous formulation to the affected skin.

Preferably, the skin condition is an inflammatory and/or chronic skin disease selected from the group consisting of rosacea, acne, xerosis, tinea pedis, eczema, allergic rushes, pruritus, tinea, psoriasis, pustulosis palmaris et plantaris, and combinations thereof.

The formulation of the invention can be used to treat, for example, acne or rosacea by applying the formulation to affected areas of the skin, such as on the face, neck, back, and chest. The formulation is preferably applied one or more times daily, weekly, bi-weekly, tri-weekly, monthly for a time sufficient to ameliorate or substantially eliminate the signs of skin conditions, such as acne or rosacea.

Preferably, the formulation is a leave-on formulation. The term "leave-on" means that the formulation is in contact with the affected skin for at least 6 hours before being removed.

In a fourth aspect, the present invention relates to a non-therapeutic method for cosmetically treating the skin, the method comprising the step of applying the topical formulation as defined herein.

It has been found that application of the formulation as defined herein by applying the formulation to the affected skin the appearance of the skin can be improved. For example, when the formulation is applied to the red areas associated with acne and rosacea, the red areas fade and a healthy skin appearance is observed.

Preferably, the non-therapeutic method for cosmetically treating the skin comprises the steps of:
1. preparing the affected skin, preferably by microneedling or exfoliation,
2. applying the formulation according to the invention to the prepared skin.

It has been found that by suitably preparing the affected parts of the skin by exfoliation or by application of a microneedle, a particularly effective cosmetic treatment method is achieved.

Preferably, the non-therapeutic method according to the present invention comprises a step of leaving the formulation on the skin for at least 6 hours, at least 12 hours, at least 1 day, at least 2 days, at least 3 days, at least 4 days, at least 5 days, at least 6 days at least 7 days at least 8 days before removing the formulation from the skin.

Removing the formulation is preferably carried out by applying water directly to the skin or via an applicator such as a cotton bud, cotton pad swob, towel, cloth, sponge, gauze, flannel, facecloth or similar.

In a preferred embodiment, the non-therapeutic method for cosmetically treating the skin comprises the steps of:
1. preparing the affected skin, preferably by microneedling or exfoliation,
2. applying the formulation according to the invention as defined herein to the prepared skin.
3. leaving the formulation in contact with the skin for between 0.25 to 8 days, preferably between 1 and 7 days, more preferably between 3 and 6 days before removing the formulation from the skin.

In a non-claimed aspect, the present disclosure relates to a kit, said kit comprising:
i) a container comprising an aqueous formulation according to the present invention,
ii) an instruction leaflet comprising instructions for a non-therapeutic method for cosmetically treating the skin with the aqueous formulation contained in the container comprising the steps of:
   1. preparing the affected skin, preferably by microneedling or exfoliation,
   2. applying the formulation contained in the container to the prepared skin,
   3. leaving the formulation in contact with the skin for between 0.25 to 8 days, preferably between 1 and 7 days, more preferably between 3 and 6 days before removing the formulation from the skin.

In a non-claimed aspect, the present disclosure relates to a kit, said kit comprising:
i) a container comprising an aqueous formulation for the topical treatment of skin conditions, comprising:
   a) 2-30 parts by weight undissolved borax particles;
   b) 0.01-10 parts by weight undissolved sulphur particles; and
   c) 20-95 parts by weight water, wherein the ratio of undissolved borax particles to undissolved sulphur particles is in the range of 20:1 to 1.5:1 at 20 °C,
ii) an instruction leaflet comprising instructions for a non-therapeutic method for cosmetically treating the skin with the aqueous formulation contained in the container comprising the steps of:
   1. preparing the affected skin, preferably by microneedling or exfoliation,
   2. applying the formulation contained in the container to the prepared skin,
   3. leaving the formulation in contact with the skin for between 0.25 to 8 days, preferably between 1 and 7 days, more preferably between 3 and 6 days before removing the formulation from the skin.

The container may be a vial, ampule, bottle or other suitable receptacle for containing the composition. The container may comprise means for opening the container such as a screw cap, snap line in the case of ampules, puncture seal or other such means for opening the container so that the composition can be transferred from the container to the applicator. The kit may further comprise an applicator such as a swob for applying the composition to the skin.

The invention will be illustrated by the following non-limiting examples

### EXAMPLES

### Example 1

A topical formulation according to the invention for use in the cosmetic treatment of rosacea was prepared according to the composition shown in Table 1:

**Table 1**

| | **Component** | Sample 1 (g) |
|---|---|---|
| Solids | Borax¹ powder | 10 |
| | Menthol | 1 |
| | Sulphur² | 3 |
| Aqueous phase | Demineralized water | 60 |
| | Ethanol (30%) | 25 |
| | Camphor | 1 |

| | | |
|---|---|---|
| ¹ Borax decahydrate EtiMaden®, ≥99.9%. Particle size: 0.063 mm (>30%) to 1.1 mm. ² Sulfur - powder Sigma Aldrich®, 99.98%. Particle size: 0.1 µm. | | |

The borax, menthol powder (Sigma Aldrich M2772) and sulphur were combined and added to the aqueous phase (water, ethanol and camphor) in a 100 mL glass bottle and vigorously shaken by hand for 10 minutes at 25 °C.

### Example 2

The formulations prepared as described in Example 1 were tested on subjects suffering from typical symptoms of rosacea, namely flushing, raised red patches ("plaques"), pimples, burning or stinging and swelling. All the subjects have previously tried to treat or ameliorate their rosacea symptoms with no effect.

The composition was applied topically according to the following method:
1. cleaning the affected area with a skin cleansing cream or lotion;
2. micro needling or exfoliating the skin lesions superficially;
3. extruding the excess secretions from the pricked skin lesions by, e.g., applying pressure adjacently to the skin lesions using fingers;
4. drying the excess with a pad;
5. gently applying the composition prepared as described in Example 1 onto the skin using an cotton pad.

The treated area was not washed nor received any further product for at least 6 h.

The method comprising steps 1-5 above was repeated daily for an average of 2 weeks.

All subjects treated with formulation 1 reported a significant (>90%) amelioration of the rosacea symptoms and overall skin condition after applying the composition according to the invention. From these, 95% have not reported recurrence of the disease for the following 6 months.

### Example 3 - Comparative example

**Table 2**

| **Component** | Sample A (g) |
|---|---|
| Borax¹ powder | 10 |
| Menthol | 1 |
| Sulphur² | 3 |
| Demineralized water | 60 |
| Ethanol (30%) | 25 |
| Camphor | 1 |

| | |
|---|---|
| ¹ Borax decahydrate EtiMaden®, ≥99.9%. Particle size: 0.063 mm (>30%) to 1.1 mm. ² Sulfur - powder Sigma Aldrich®, 99.98%. Particle size: 0.1 µm. | |

The borax, menthol powder (Sigma Aldrich M2772) and sulphur were combined and added to the demineralized water in a vessel and heated to 80°C for 15 minutes with stirring. The resultant solution was cooled to room temperature, and added to ethanol and camphor in a 100 mL glass bottle and vigorously shaken by hand for 10 minutes at 25 °C.

Subjects treated with the formulation according to the comparative example (A) did not see a reduction in symptoms associated with rosacea.

### Example 4

A topical formulation according to the invention for use in the cosmetic treatment of rosacea was prepared according to the composition shown in Table 3:

**Table 3**

| | **Component** | Sample B (g) |
|---|---|---|
| Solids | Borax¹ powder | 3 |
| | Menthol | 1 |
| | Sulphur² | 20 |
| Aqueous phase | Demineralized water | 50 |
| | Ethanol (30%) | 25 |
| | Camphor | 1 |

| | | |
|---|---|---|
| ¹ Borax decahydrate EtiMaden®, ≥99.9%. Particle size: 0.063 mm (>30%) to 1.1 mm. ² Sulfur - powder Sigma Aldrich®, 99.98%. Particle size: 0.1 µm. | | |

The borax, menthol powder (Sigma Aldrich M2772) and sulphur were combined and added to the aqueous phase (water, ethanol and camphor) in a 100 mL glass bottle and vigorously shaken by hand for 10 minutes at 25 °C.

Sample B has an unpleasant smell. Subjects treated with samples B do not see a reduction in symptoms associated with rosacea.

### Example 5

A clinical evaluation of a topical formulation according to Table 1 and a topical formulation according to Table 3 is being carried out. A first group of patients will be treated with the topical formulation according to Table 1 and second group of patients will be treated with the a control composition. The visual appearance of the patients' face will be recorded prior to treatment, after 1 day, 5 days, 15 days and 30 days.

## Claims

1. An aqueous formulation for the topical treatment of skin conditions, comprising:
a) 2-30 parts by weight undissolved borax particles;
b) 0.01-10 parts by weight undissolved sulphur particles; and
c) 20-95 parts by weight water, wherein the ratio of undissolved borax particles to undissolved sulphur particles is in the range of 20:1 to 1.5:1 at 20 °C.

2. Aqueous formulation according to claim 1, wherein the ratio of undissolved borax particles to undissolved sulphur particles is in the range of 15:1 to 2:1, preferably 12:1 to 3:1, more preferably 10:1 to 4:1.

3. Aqueous formulation, according to claim 1 or 2, wherein at least 80 wt.% of the undissolved borax particles has a particle size of between 50-3000 µm, preferably 100-1200 µm, more preferably 200-1000 µm.

4. Aqueous formulation according to anyone of the preceding claims, wherein at least 80 wt.% of the undissolved sulphur particles has a particle size of 0.0050-5 µm, preferably 0.010-1 µm, more preferably 0.05-0.5 µm.

5. Aqueous formulation according to anyone of the preceding claims, wherein the aqueous formulation comprises 5-50 parts by weight of a water miscible organic solvent, preferably wherein the combination of water and water miscible organic solvent constitutes at least 60 wt.%, preferably at least 75 wt.% of the aqueous formulation.

6. Aqueous formulation according to claim 5, wherein the water miscible organic solvent is selected from the group consisting of ethanol, ethylene glycol propylene glycol, glycerol and mixtures thereof.

7. Aqueous formulation according to anyone of the preceding claims, wherein the combination of undissolved borax particles, undissolved sulphur particles, water and water miscible organic solvent constitutes at least 80 wt.%, preferably at least 90 wt.% of the aqueous formulation.

8. Aqueous formulation according to anyone of the preceding claims, wherein the formulation comprises 0.001-5 parts by weight dermatologically acceptable terpene selected from the group consisting of menthol, camphor, pinene, thujone, limone and mixtures thereof.

9. Aqueous formulation according to anyone of the preceding claims, wherein the aqueous formulation is liquid or semi-solid at 20°C.

10. An aqueous formulation according to anyone of the preceding claims, comprising based on total weight of the formulation:
a) 5-15 wt.% undissolved borax particles;
b) 0.1-10 wt.% undissolved sulphur particles;
c) 50-70 wt.% water; and
d) 5-35 wt.% water miscible organic solvent, wherein the ratio of solids (undissolved borax particles : undissolved sulphur particles) is at least 20:1 to 1.5:1 at 20°C.

11. Method for preparing an aqueous formulation according to anyone of the preceding claims, comprising mixing borax particles, sulphur particles, water and the optional water miscible organic solvent at a temperature of not more than 30 °C.

12. An aqueous formulation for use in the treatment of a skin condition, said use comprising the step of applying the aqueous formulation to the affected skin, wherein the aqueous formulation is an aqueous formulation according to any one or more of claims 1-10, or obtainable by a method according to claim 11.

13. Aqueous formulation for use in the treatment of a skin condition according to claim 12, wherein the skin condition is an inflammatory and/or chronic skin disease selected from the group consisting of rosacea, acne, xerosis, tinea pedis, eczema, allergic rushes, pruritus, tinea, psoriasis, pustulosis palmaris et plantaris, and combinations thereof.

14. A non-therapeutic method for cosmetically treating the skin, the method comprising the step of applying the aqueous formulation according to anyone of claims 1-10 or obtainable by the method according to claim 11 to the affected skin.

15. A non-therapeutic method for cosmetically treating the skin according to claim 14, the method comprising the steps of:
i. preparing the affected skin, preferably by microneedling or exfoliation,
ii. applying the formulation according to anyone of claims 1-10 or obtainable by the method according to claim 11 to the prepared skin.

## Patentansprüche

1. Wässrige Formulierung zur topischen Behandlung von Hautprobleme, umfassend:
a) 2 - 30 Gewichtsanteile ungelöste Boraxteilchen;
b) 0,01 - 10 Gewichtsanteile ungelöste Schwefelteilchen; und
c) 20 - 95 Gewichtsanteile Wasser, wobei das Verhältnis von ungelösten Boraxteilchen zu ungelösten Schwefelteilchen im Bereich von 20:1 bis 1,5:1 bei 20 °C liegt.

2. Wässrige Formulierung nach Anspruch 1, wobei das Verhältnis von ungelösten Boraxteilchen zu ungelösten Schwefelteilchen im Bereich von 15:1 bis 2:1, vorzugsweise 12:1 bis 3:1, besonders bevorzugt 10:1 bis 4:1 liegt.

3. Wässrige Formulierung nach Anspruch 1 oder 2, wobei mindestens 80 Gew.-% der ungelösten Boraxteilchen eine Teilchengröße zwischen 50 - 3000 µm, vorzugsweise 100 - 1200 µm, besonders bevorzugt 200 - 1000 µm, aufweisen.

4. Wässrige Formulierung nach einem der vorhergehenden Ansprüche, wobei mindestens 80 Gew.-% der ungelösten Schwefelteilchen eine Teilchengröße von 0,0050 - 5 µm, vorzugsweise 0,010 - 1 µm, besonders bevorzugt 0,05 - 0,5 µm aufweisen.

5. Wässrige Formulierung nach einem der vorhergehenden Ansprüche, wobei die wässrige Formulierung 5 - 50 Gewichtsanteile eines mit Wasser mischbaren organischen Lösungsmittels umfasst, vorzugsweise wobei die Kombination aus Wasser und mit Wasser mischbarem organischem Lösungsmittel mindestens 60 Gew.-%, vorzugsweise mindestens 75 Gew.-% der wässrigen Formulierung ausmacht.

6. Wässrige Formulierung nach Anspruch 5, wobei das mit Wasser mischbare organische Lösungsmittel ausgewählt ist aus der Gruppe bestehend aus Ethanol, Ethylenglykol, Propylenglykol, Glycerin und Mischungen davon.

7. Wässrige Formulierung nach einem der vorhergehenden Ansprüche, wobei die Kombination aus ungelösten Boraxteilchen, ungelösten Schwefelteilchen, Wasser und mit Wasser mischbarem organischem Lösungsmittel mindestens 80 Gew.-%, vorzugsweise mindestens 90 Gew.-% der wässrigen Formulierung ausmacht.

8. Wässrige Formulierung nach einem der vorhergehenden Ansprüche, wobei die Formulierung 0,001 - 5 Gewichtsanteile eines dermatologisch akzeptablen Terpens, ausgewählt aus der Gruppe bestehend aus Menthol, Kampfer, Pinen, Thujon, Limone und Mischungen davon, enthält.

9. Wässrige Formulierung nach einem der vorhergehenden Ansprüche, wobei die wässrige Formulierung bei 20°C flüssig oder halbfest ist.

10. Wässrige Formulierung nach einem der vorangehenden Ansprüche, aufweisend bezogen auf das Gesamtgewicht der Formulierung:
a) 5 - 15 Gew.-% ungelöste Boraxteilchen;
b) 0,1 - 10 Gew.-% ungelöste Schwefelteilchen;
c) 50 - 70 Gew.-% Wasser; und
d) 5 - 35 Gew.-% mit Wasser mischbares organisches Lösungsmittel, wobei das Verhältnis der Feststoffe (ungelöste Boraxteilchen : ungelöste Schwefelteilchen) mindestens 20:1 bis 1,5:1 bei 20°C beträgt.

11. Verfahren zur Herstellung einer wässrigen Formulierung nach einem der vorhergehenden Ansprüche, umfassend das Mischen von Boraxteilchen, Schwefelteilchen, Wasser und dem optionalen mit Wasser mischbaren organischen Lösungsmittel bei einer Temperatur von nicht mehr als 30°C.

12. Wässrige Formulierung zur Verwendung bei der Behandlung einesHautproblemes, wobei die Verwendung den Schritt des Auftragens der wässrigen Formulierung auf die betroffene Haut umfasst, wobei die wässrige Formulierung eine wässrige Formulierung nach einem oder mehreren der Ansprüche 1 - 10 ist oder durch ein Verfahren nach Anspruch 11 erhältlich ist.

13. Wässrige Formulierung zur Verwendung bei der Behandlung eines Hautproblemes nach Anspruch 12, wobei das Hautproblem eine entzündliche und/oder chronische Hauterkrankung ist, ausgewählt aus der Gruppe bestehend aus Rosacea, Akne, Xerosis, Tinea pedis, Ekzem, allergischen Rash, Pruritus, Tinea, Psoriasis, Pustulosis palmaris et plantaris und Kombinationen davon.

14. Nicht-therapeutisches Verfahren zur kosmetischen Behandlung der Haut, wobei das Verfahren den Schritt des Auftragens der wässrigen Formulierung nach einem der Ansprüche 1 - 10 umfasst oder durch das Verfahren nach Anspruch 11 auf die betroffene Haut erhältlich ist.

15. Nicht-therapeutisches Verfahren zur kosmetischen Behandlung der Haut nach Anspruch 14, wobei das Verfahren die folgenden Schritte umfasst:
i. Vorbereiten der betroffenen Haut, vorzugsweise durch Micro-Needling oder Exfoliation,
ii. Auftragen der Formulierungnach einem der Ansprüche 1 - 10 oder erhältlich durch das Verfahren nach Anspruch 11 auf die vorbereitete Haut.

## Revendications

1. Formulation aqueuse destinée au traitement local d'affections cutanées, comprenant :
a) 2-30 parties en poids de particules de borax non dissoutes ;
b) 0,01-10 parties en poids de particules de soufre non dissoutes ; et
c) 20-95 parties en poids d'eau, dans laquelle le rapport des particules de borax non dissoutes aux particules de soufre non dissoutes est compris dans l'intervalle de 20:1 à 1,5:1 à 20°C.

2. Formulation aqueuse selon la revendication 1, dans laquelle le rapport des particules de borax non dissoutes aux particules de soufre non dissoutes est compris dans l'intervalle de 15:1 à 2:1, de préférence 12:1 à 3:1, plus préférablement 10:1 à 4:1.

3. Formulation aqueuse selon la revendication 1 ou 2, dans laquelle au moins 80% en poids des particules de borax non dissoutes a une taille des particules comprise entre 50-3000 µm, de préférence 100-1200 µm, plus préférablement 200-1000 µm.

4. Formulation aqueuse selon l'une quelconque des revendications précédentes, dans laquelle au moins 80% en poids des particules de soufre non dissoutes a une taille des particules de 0,0050-5 µm, de préférence 0,010-1 µm, plus préférablement 0,05-0,5 µm.

5. Formulation aqueuse selon l'une quelconque des revendications précédentes, ladite formulation aqueuse comprenant 5-50 parties en poids d'un solvant organique miscible à l'eau, de préférence, dans laquelle la combinaison de l'eau et du solvant organique miscible à l'eau constitue au moins 60% en poids, de préférence 75% en poids de la formulation aqueuse.

6. Formulation aqueuse selon la revendication 5, dans laquelle le solvant organique miscible à l'eau est choisi dans le groupe constitué par l'éthanol, l'éthylèneglycol, le propylèneglycol, le glycérol et leurs mélanges.

7. Formulation aqueuse selon l'une quelconque des revendications précédentes, dans laquelle la combinaison des particules de borax non dissoutes, des particules de soufre non dissoutes, de l'eau et du solvant organique miscible à l'eau constitue au moins 80% en poids, de préférence au moins 90% en poids de la formulation aqueuse.

8. Formulation aqueuse selon l'une quelconque des revendications précédentes, ladite formulation comprenant 0,001-5 parties en poids de terpène dermatologiquement acceptable, choisi dans le groupe constitué par le menthol, le camphre, le pinène, la thuyone, la limone et leurs mélanges.

9. Formulation aqueuse selon l'une quelconque des revendications précédentes, ladite formulation aqueuse étant liquide ou semi-solide à 20°C.

10. Formulation aqueuse selon l'une quelconque des revendications précédentes, comprenant, sur la base du poids total de la formulation :
a) 5-15% en poids de particules de borax non dissoutes ;
b) 0,1-10% en poids de particules de soufre non dissoutes ;
c) 50-70% en poids d'eau ; et
d) 5-35% en poids de solvant organique miscible à l'eau, dans laquelle le rapport des solides (particules de borax non dissoutes : particules de soufre non dissoutes) est d'au moins 20:1 à 1,5:1 à 20°C.

11. Procédé de préparation d'une formulation aqueuse selon l'une quelconque des revendications précédentes, comprenant le mélange des particules de borax, des particules de soufre, de l'eau et du optioneel solvant organique miscible à l'eau à une température ne dépassant pas les 30°C.

12. Formulation aqueuse destinée à être utilisée dans le traitement d'une affection cutanée, ladite utilisation comprenant l'étape consistant à appliquer la formulation aqueuse sur la peau affectée, ladite formulation aqueuse étant une formulation aqueuse selon l'une quelconque ou plusieurs des revendications 1-10, ou pouvant être obtenue par un procédé selon la revendication 11.

13. Formulation aqueuse destinée à être utilisée dans le traitement d'une affection cutanée selon la revendication 12, dans laquelle l'affection cutanée est une maladie inflammatoire et/ou chronique de la peau choisie dans le groupe constitué par la rosacée, l'acné, la xérose, la tinea pedis, l'eczéma, les rashes allergiques, le prurit, la tinea, le psoriasis, la pustulose palmaire et plantaire, et leurs combinaisons.

14. Procédé non thérapeutique de traitement cosmétique de la peau, ledit procédé comprenant l'étape d'application de la formulation aqueuse selon l'une quelconque des revendications 1-10 ou pouvant être obtenue par le procédé selon la revendication 11 sur la peau affectée.

15. Procédé non thérapeutique de traitement cosmétique de la peau selon la revendication 14, ledit procédé comprenant les étapes de :
i. préparation de la peau affectée, de préférence par microneedling ou exfoliation,
ii. application de la formulation selon l'une quelconque des revendications 1-10 ou pouvant être obtenue par le procédé selon la revendication 11 sur la peau préparée.
